# EUROPEAN PATENT SPECIFICATION

(11) **EP 4 054 663 B1**
(45) Date of publication and mention of the grant of the patent: **30.04.2025**
(21) Application number: 20816770.0
(22) Date of filing: 05.11.2020
(51) Int. Cl.: A61L 29/04, A61M 25/00

(54) **FLUSHABLE CATHETER EXTENSIONS**
SPÜLBARE KATHETERVERLÄNGERUNGEN
EXTENSIONS DE CATHÉTER RINÇABLES

(30) Priority: 06.11.2019 US 201962931596 P
(43) Date of publication of application: 14.09.2022
(73) Proprietor: Hollister Incorporated, Libertyville, IL 60048 (US)
(72) Inventor: SILEIKA, Tadas, S., Libertyville, IL 60048 (US); DEAN, Nicole, L., Libertyville, IL 60048 (US); MCNULTY, Vivienne, Libertyville, IL 60048 (US)
(74) Representative: FRKelly
(86) International application number: PCT/US2020/059217
(87) International publication number: WO 2021/092253

(56) References cited:
- EP-A2- 3 079 749
- EP-A2- 3 079 751
- WO-A1-2014/077886

## Description

### Field of the Disclosure

The present disclosure is generally related to intermittent catheters for use in draining urine from the human bladder and, more particularly, to intermittent urinary catheter assemblies for use by those facing catheter drainage issues such as wheelchair bound users and the like.

### Background of the Disclosure

Intermittent catheterization is a good option for many users who suffer from various abnormalities of the urinary system. A common situation is where a single use, individually packaged, sterile catheter is used. An important criterion for any single use product is that it be user friendly, as patients ultimately will perform self-catheterization outside of the clinical setting and without trained medical personnel to assist.

Due to the advancements that have been made in intermittent urinary catheter products, it is now quite common for users who require catheterization on a recurring basis to perform this procedure themselves. The intermittent urinary catheter products, which are currently available, render the catheterization procedure straightforward for many catheter users. However, this is not the case for some users who may encounter difficulty in being able to discharge urine from an intermittent urinary catheter directly into a toilet or a urine collection bag.

For such users who would otherwise be able to administer self-intermittent catheterization for voiding their bladder of urine multiple times per day, existing intermittent urinary catheter products have not provided a suitable solution for discharging urine from the catheter into a toilet, especially for allowing the users to be in close proximity to the toilet during the catheterization procedure without concern for urine spillage. The document EP 3 079 751 A2 discloses a catheter drainage accessory according to the preamble of claim 1.

### Summary of the Disclosure

There are several aspects of the present subject matter, which may be embodied separately or together in the catheter drainage accessory devices and systems described and claimed below.

In an exemplary embodiment, a catheter drainage accessory includes a connector, such as a nipple or fitting, that is adapted to be inserted into the drainage member of a urinary catheter and a urine drainage conduit that may be extended away from the drainage member to a receptacle, e.g., a toilet. The catheter drainage accessory being made from a flushable material.

### Brief Description of the Drawings

Fig. 1 is a perspective view of a first embodiment of a catheter drainage accessory;
Fig. 2 is a perspective view of another embodiment of a catheter drainage accessory; and
Fig. 3 a partial cross-sectional view of a catheter drainage accessory, wherein a portion of the accessory having cavities or voids.

### Detailed Description of the Disclosure

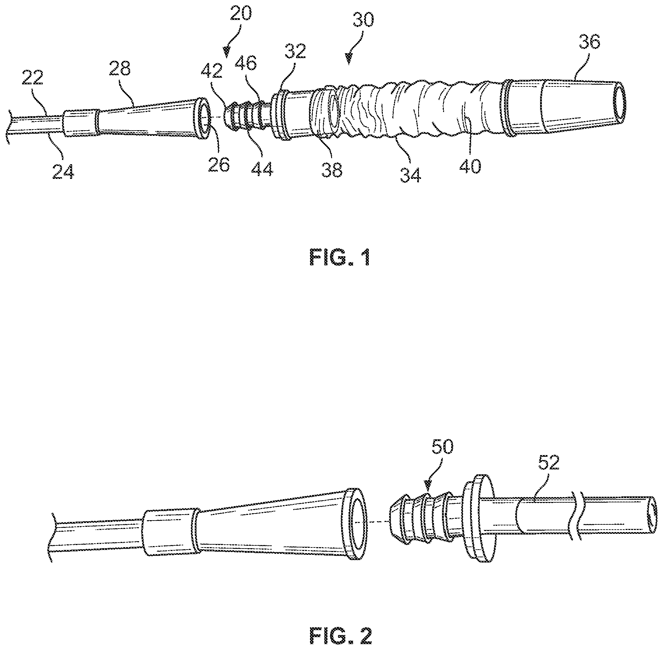

Referring to Fig. 1, an intermittent urinary catheter assembly 20 includes catheter tube 22 having a proximal insertion end (not shown) and a distal end 24 which is located remote from the proximal insertion end. The catheter tube 22 has a lumen (not shown) which extends from at or near the proximal insertion end to the distal end 24 to accommodate the draining of urine from a human bladder. The catheter tube 22 also has at least one drainage opening (not shown) which extends through the catheter tube 22 to the lumen in a location which is at or near the proximal insertion end. The catheter tube 22 also has a discharge opening 26 associated with the distal end 24 thereof. The discharge opening may be defined by a drainage member 28, such as the illustrated funnel, to permit urine voided from a human bladder to be discharged therefrom.

The catheter assembly 22 also includes a drainage accessory 30 that is operatively attachable to the catheter tube 22. The drainage accessory may be a drainage extension or may be extendible during use. The drainage accessory 30 may include a connector 32 (such as a fitting or nipple), a drainage conduit (such as a sleeve 34) and, optionally, a drainage funnel 36. In the illustrated embodiment, the urine drainage sleeve 34 has a first end 38 secured to the connector 32 and a second, extendable end 40 secured to the drainage funnel 36. The connector 32 may be, for example, a Christmas tree fitting which can be inserted and connected to the opening 26 defined by drainage member 28.

The sleeve 34 may be flexible so as to provide for a flexible and compact, when packaged, fluid path solution. The sleeve's 34 relative surface area may be relatively high when compared to the volume of the material that the sleeve is made from. In one embodiment, the thickness of the sleeve 34 may be 0.02 mm.

Because the drainage member 28 is typically formed of a rubber or rubber-like material, and as a result of the increasing diameter portions 42, 44, 46, etc. of the Christmas tree fitting of connector 32, the user can very quickly and easily insert the Christmas tree fitting into the drainage member 28 until it is secured at which point the urine drainage sleeve 34 can be extended into a deployed configuration for use by pulling on the drainage funnel 36.

Referring to Fig. 2, the drainage accessory 50 shown therein has some similar features to that of accessory 30 of Fig. 1, with the exception that the drainage conduit is a drainage tube 52.

The drainage accessories 30 and 50 may be made of a flushable material. For example, one or more of the connector, fluid conduit (sleeve/tube) and the funnel may be made of a flushable material. Such materials may be materials that are affected by a fluid (for example, water, urine, or fluids utilized in toilet and plumbing systems). Such materials may be water disintegratable or disintegrable materials. As used herein, the terms "water disintegratable" and "water disintegrable" refer to materials that are water soluble, water degradable, or water hydrolysable, and which dissolve, degrade, or otherwise break down when in contact with water over a selected period of time. In other embodiments, the material may be enzymatically hydrolysable. The water disintegrable and enzymatically hydrolysable materials are preferably flushable materials which are suitable for disposal in a toilet or sanitary system and, even more preferably, biodegradable flushable materials which may be chemically broken down by living organisms or other biological means.

Such water disintegrable or enzymatically hydrolysable materials may include, for example, polyvinyl alcohol, including but not limited to an extrudable polyvinyl alcohol, polyacrylic acids, polyactic acid, polyesters, polyglycolide, polyglycolic acid, poly lactic-co-glycolic acid, polylactide, amines, polyacrylamides, poly(N-(2-Hydroxypropyl) methacrylamide), starch, modified starches or derivatives, amylopectin, pectin, xanthan, scleroglucan, dextrin, chitosans, chitins, agar, alginate, carrageenans, laminarin, saccharides, polysaccharides, sucrose, polyethylene oxide, polypropylene oxide, acrylics, polyacrylic acid blends, poly(methacrylic acid), polystyrene sulfonate, polyethylene sulfonate, lignin sulfonate, polymethacrylamides, copolymers of aminoalkyl-acrylamides and methacrylamides, melamine-formaldehyde copolymers, vinyl alcohol copolymers, cellulose ethers, poly-ethers, polyethylene oxide, blends of polyethylene-polypropylene glycol, carboxymethyl cellulose, guar gum, locust bean gum, hydroxyproply cellulose, vinylpyrrolidone polymers and copolymers, polyvinyl pyrrolidone-ethylene-vinyl acetate, polyvinyl pyrrolidone-carboxymethyl cellulose, carboxymethyl cellulose shellac, copolymers of vinylpyrrolidone with vinyl acetate, hydroxyethyl cellulose, gelatin, poly-caprolactone, poly(p-dioxanone), or combinations, blends, or co-polymers of any of the above materials. The water disintegrable materials may also be any of those that are included in certified flushable products that meet the National Sanitation Foundation standards for flushability or materials and products that meet INDA/EDANA Flushability Guidelines or the UK Water Industry Research test protocols set forth in "Test Protocol to Determine the Flushability of Disposable Products, Review of the Manufactures 3^{rd} Ed. Guidance Document," 2013, by Drinkwater et al. While catheters made from water disintegrable materials may be disposed of in a toilet, it is not necessary to dispose of such catheters in a toilet and such catheters may also be disposed in normal municipal waste systems or garbage collection systems.

In one embodiment, the materials may include starches, complex carbohydrates, polylactic acid, polyhydroxyalkanoate, polyanhydrides (such as copolymers of 1, 3-bis(p-carboxyphenoxy)propane and sebacic acid), polyesters (such as poly(lactic/glycolic acid)).

In one embodiment, the funnel connector, drainage conduit, and the drainage funnel can be made out of starch materials, such as raw pasta (i.e., macaroni and noodle products). Uncooked, raw pasta has been utilized in an attempt to limit the use of plastic drinking straws. Uncooked pasta can hold up to fluid transfer for up to an hour, which is far longer than what is needed to pass urine through the IC. Thereby, the more rigid components and elements of the accessory, such as the funnel connector and the funnel, can be extruded and made from pasta, and bound to the fluid transfer conduit (sleeve/tube) with starch-based adhesives, while maintaining surface features such as the grip on the funnel and the funnel connector.

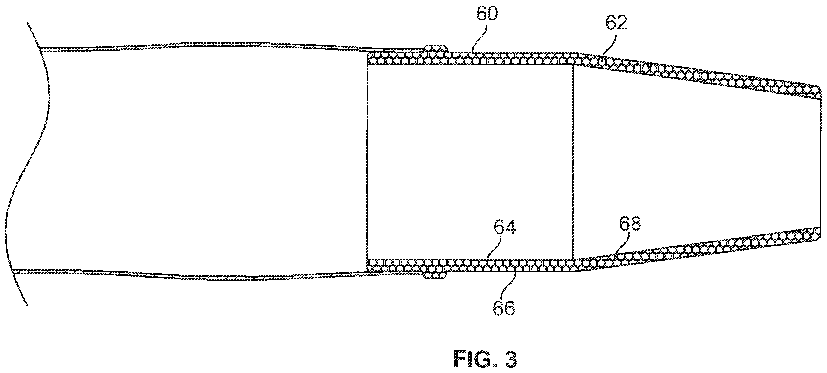

Furthermore, the drainage accessories may have a structure that is conducive for degrading or disintegrating the drainage accessory in water. In one embodiment, at least a portion of the drainage accessory has such a structure. For example, one or more of the connector, sleeve/conduit and drainage funnel may have such a structure. In one embodiment, the structure of the drainage accessory may be made so as to enhance the size of the surface area that can come into contact with water. For example, the connector, sleeve/conduit and/or drainage funnel may have one or more interior voids. As the accessory breaks down and water enters the void(s), the water will then also come into contact with the inner walls forming the void(s). In one embodiment, the portion of the drainage accessory may include an outer shell with an interior void. In other embodiments, the portion may include a plurality of voids or plurality of interior walls and voids. For example, Fig. 3 illustrates an exemplary embodiment wherein the drainage funnel 60 includes one or more interior voids 62. The internal voids are between or within walls 64 and 66. Furthermore, when there are a plurality of voids, the plurality of voids may be separated by interior walls 68. In this embodiment, the interior of the drainage funnel 60 includes honeycombs.

Upon flushing, the entire drainage accessory assembly will degrade in water over the course of 24-96 hours.

## Claims

1. A catheter drainage accessory (30) adapted to be connected to a urinary catheter, comprising:
a connector (32) comprising a nipple or fitting configured to be operably connected to a drainage member (28) of a urinary catheter;
a drainage conduit (34) for draining urine from the accessory (30) into a waste receptacle;
wherein at least one of the connector and drainage conduit is made of a water flushable material; and
**characterised in that** at least a portion of the drainage accessory made from the flushable material includes an outer shell and the outer shell includes at least a first wall (66) and a second wall (64), and one or more voids (62) being between the first wall (66) and the second wall (64).

2. The catheter accessory of claim 1, further including a drainage funnel (36, 60) wherein the drainage conduit (34) has a first end (38) secured to the connector (32) and a second end secured (40) to the drainage funnel (36, 60).

3. The accessory of any one of claims 1-2, wherein the water flushable material is made from a starch.

4. The accessory of any one of claims 1-3, wherein the water flushable material is made from a complex carbohydrate.

5. The accessory of any one of claim 1-4, wherein the water flushable material is made of one or more of polylactic acid, polyhydroxyalkanoate, polyanhydrides, and polyesters.

6. The accessory of any one of claims 1-5, wherein the water flushable material is made from one or more of copolymers of 1, 3-bis(p-carboxyphenoxy)propane and sebacic acid, and poly(lactic/glycolic acid)).

7. The accessory of any one of claims 1-6, wherein the water flushable material is made from raw pasta.

8. The accessory of claim 1, wherein the one or more inner voids (62) comprise a plurality of voids (62).

9. The accessory of claim 8, wherein the plurality of inner voids (62) are separated by inner walls (68).

10. The accessory of any one of claims 8-9, wherein the inner voids (62) are generally honeycomb shaped.

11. The accessory of any one of claims 1-10, wherein the drainage conduit (34) comprises a sleeve.

12. The accessory of claim 11, wherein the sleeve is flexible.

## Patentansprüche

1. Katheterdrainagezubehörteil (30), das dazu angepasst ist, mit einem Harnkatheter verbunden zu sein, umfassend:
einen Verbinder (32), der einen Nippel oder ein Fitting umfasst, der/das dazu konfiguriert ist, mit einem Drainageelement (28) eines Harnkatheters in Wirkverbindung zu stehen;
eine Drainageleitung (34) zum Ablassen von Urin aus dem Zubehörteil (30) in einen Abfallbehälter;
wobei der Verbinder und mindestens eines des Verbinders und der Drainageleitung aus einem wasserspülbaren Material hergestellt ist; und
**dadurch gekennzeichnet, dass**
mindestens ein Teil des Drainagezubehörs, das aus dem spülbaren Material hergestellt ist, einen äußeren Mantel beinhaltet und der äußere Mandel mindestens eine erste Wand (66) und eine zweite Wand (64) beinhaltet und sich ein oder mehrere Leerräume (62) zwischen der ersten Wand (66) und der zweiten Wand (64) befinden.

2. Katheterzubehörteil nach Anspruch 1, das weiter einen Drainagetrichter (36, 60) beinhaltet, wobei die Drainageleitung (34) ein erstes Ende (38) aufweist, das an dem Verbinder (32) befestigt ist, und ein zweites Ende (40), das an dem Drainagetrichter (36, 60) befestigt ist.

3. Zubehörteil nach einem der Ansprüche 1-2, wobei das wasserspülbare Material aus einer Stärke hergestellt ist.

4. Zubehörteil nach einem der Ansprüche 1-3, wobei das wasserspülbare Material aus einem komplexen Kohlenhydrat hergestellt ist.

5. Zubehörteil nach einem der Ansprüche 1-4, wobei das wasserspülbare Material aus einem oder mehreren von Polymilchsäure, Polyhydroxyalkanoat, Polyanhydriden und Polyestern hergestellt ist.

6. Zubehörteil nach einem der Ansprüche 1-5, wobei das wasserspülbare Material aus einem oder mehreren von Copolymeren von 1, 3-Bis(p-carboxyphenoxy)propan und Sebacinsäure und Poly(milchsäure/glykolsäure) hergestellt ist.

7. Zubehörteil nach einem der Ansprüche 1-6, wobei das wasserspülbare Material aus Rohteigwaren hergestellt ist.

8. Zubehörteil nach Anspruch 1, wobei der eine oder die mehreren inneren Leerräume (62) eine Vielzahl von Leerräumen (62) umfassen.

9. Zubehörteil nach Anspruch 8, wobei die Vielzahl innerer Leerräume (62) durch Innenwände (68) getrennt ist.

10. Zubehörteil nach einem der Ansprüche 8-9, wobei die inneren Leerräume (62) im Allgemeinen wabenförmig sind.

11. Zubehörteil nach einem der Ansprüche 1-10, wobei die Drainageleitung (34) eine Hülle umfasst.

12. Zubehörteil nach Anspruch 11, wobei die Hülle flexibel ist.

## Revendications

1. Accessoire de drainage de cathéter (30) adapté pour être relié à un cathéter urinaire, comprenant :
un raccord (32) comprenant un embout ou un élément de fixation configuré pour être relié de manière fonctionnelle à un élément de drainage (28) d'un cathéter urinaire ;
un conduit de drainage (34) permettant l'évacuation de l'urine depuis l'accessoire (30) vers une poubelle ;
dans lequel au moins l'un du raccord et du conduit de drainage est fabriqué à partir d'un matériau rinçable ; et
**caractérisé en ce que**
au moins une partie de l'accessoire de drainage fabriqué à partir du matériau rinçable comporte une coque extérieure et la coque extérieure comporte au moins une première paroi (66) et une seconde paroi (64), et un ou plusieurs espaces (62) se situant entre la première paroi (66) et la seconde paroi (64).

2. Accessoire de cathéter selon la revendication 1, comportant également un entonnoir de drainage (36, 60) dans lequel le conduit de drainage (34) présente une première extrémité (38) fixée au raccord (32) et une seconde extrémité fixée (40) à l'entonnoir de drainage (36, 60).

3. Accessoire selon l'une quelconque des revendications 1 à 2, dans lequel le matériau rinçable est fabriqué à partir d'un amidon.

4. Accessoire selon l'une quelconque des revendications 1 à 3, dans lequel le matériau rinçable est fabriqué à partir d'un glucide complexe.

5. Accessoire selon l'une quelconque des revendications 1 à 4, dans lequel le matériau rinçable est fabriqué à partir d'un ou plusieurs éléments parmi de l'acide polylactique, du polyhydroxyalcanoate, des polyanhydrides et des polyesters.

6. Accessoire selon l'une quelconque des revendications 1 à 5, dans lequel le matériau rinçable est fabriqué à partir d'un ou plusieurs éléments parmi des copolymères de 1,3-bis(p-carboxyphénoxy)propane et d'acide sébacique, et d'acide poly(lactique/glycolique)).

7. Accessoire selon l'une quelconque des revendications 1 à 6, dans lequel le matériau rinçable est fabriqué à partir de pâtes alimentaires crues.

8. Accessoire selon la revendication 1, dans lequel les un ou plusieurs espaces (62) intérieurs comprennent une pluralité d'espaces (62).

9. Accessoire selon la revendication 8, dans lequel la pluralité d'espaces (62) intérieurs sont séparés par des parois intérieures (68).

10. Accessoire selon l'une quelconque des revendications 8 à 9, dans lequel les espaces (62) intérieurs sont généralement en forme de nid d'abeille.

11. Accessoire selon l'une quelconque des revendications 1 à 10, dans lequel le conduit de drainage (34) comprend un manchon.

12. Accessoire selon la revendication 11, dans lequel le manchon est flexible.
